# EUROPEAN PATENT SPECIFICATION

(11) **EP 4 706 750 B1**
(45) Date of publication and mention of the grant of the patent: **18.11.2026**
(21) Application number: 26154546.1
(22) Date of filing: 27.08.2024
(51) Int. Cl.: A61N 1/372, A61N 1/375, A61N 1/05

(54) **MODULAR BIOSTIMULATOR**
MODULARER BIOSTIMULATOR
BIOSTIMULATEUR MODULAIRE

(30) Priority: 30.08.2023 US 202363535391 P; 23.08.2024 US 202418814373
(43) Date of publication of application: 11.03.2026
(62) Document number(s) of the earlier application(s) in accordance with Art. 76 EPC: 24196666.2 / 4 516 348
(73) Proprietor: Pacesetter, Inc., Sylmar, CA 91342 (US)
(72) Inventor: WISE, Derek, Sylmar, CA 91342 (US); ARNAR, Bernhard, Sylmar, CA 91342 (US); SERVI, Andy, Sylmar, CA 91342 (US)
(74) Representative: Barker Brettell Sweden AB

(56) References cited:
- WO-A1-2022/267718
- US-A1- 2019 290 323
- US-A1- 2023 012 417
- US-A1- 2023 050 125

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch (LBB) is an alternative to His-bundle pacing. Pacing at the LBB involves pacing past the His-bundle toward the right ventricular apex. More particularly, a pacing site for LBB pacing is typically below the His-bundle, on the interventricular septal wall. To achieve optimal results, the pacing site for physiological pacing at the LBB can be high on the interventricular septal wall, in the region close to the tricuspid valve and pulmonary artery outflow track. Furthermore, the pacing site may be at a depth of up to 1 cm within the septal wall.

US 2023/0012417 A1 discloses a medical system including an implantable medical device configured to be positioned within an atrium of a heart. The implantable medical device includes a housing carrying a return electrode, a first leadlet, a second leadlet, and a fixation device. The medical system may be configured to deliver a variety of therapies, including one or more of ventricle-from-atrium cardiac therapy, left bundle branch pacing therapy, or cardiac resynchronization therapy.

### SUMMARY

The pacing location and pacing depth required for effective left bundle branch (LBB) pacing can be challenging to achieve. Existing leadless pacemakers may not fit, or may interfere with heart structures, when being placed at the optimal pacing site. More particularly, there is limited volume to deliver a leadless pacemaker into an upper ventricular area having the optimal pacing site. Existing leadless pacemakers have bodies that are long and rigid and, when maneuvered to the interventricular septal wall, could extend into contact with the cardiac tissue of a ventricular free wall or the tricuspid valve during contraction of the heart. Achieving acceptable positioning of a leadless pacemaker may require several approach attempts. Thus, there is a need for a leadless biostimulator that can be maneuvered, potentially several times, to the interventricular septal wall without interfering with adjacent structures of the heart.

A biostimulator is described. In an embodiment, the biostimulator is a modular biostimulator and includes a header module and a housing module. The header module includes a pacing electrode, and a header leadlet electrically connected to the pacing electrode. The housing module includes pacing circuitry electrically connected to a power source, and a housing leadlet electrically coupled to the pacing circuitry. The biostimulator also comprises a cable connector interconnecting the housing leadlet and the header leadlet. The cable connector is configured to provide a wet connection between the housing module and the header module.

In an embodiment, the biostimulator comprises an attachment mechanism to mechanically couple the header to the housing module.

In an embodiment, the attachment mechanism includes one or more tabs to engage one or more slots.

In an embodiment, the housing module includes a guide lumen radially offset from a housing axis of the housing module.

In an embodiment, the biostimulator further comprises a guide rail coupled to the header module. In this embodiment, the guide rail extends through the guide lumen of the housing module.

In an embodiment, the electrical socket is within the guide lumen.

In embodiments, the fixation element includes a helical anchor or fixation tines (1502).

A biostimulator system is described. In an embodiment, the biostimulator system includes a biostimulator transport system. The biostimulator of the disclosure is mounted on the biostimulator transport system.

The biostimulator transport system can deliver the biostimulator to or from a target anatomy.

In an embodiment, the biostimulator transport system includes a guide rail and/or a guide shaft coupled to the header module.

In an embodiment, the guide shaft is electrically coupled to the pacing electrode.

### BRIEF DESCRIPTION OF THE DRAWINGS

The novel features of the invention are set forth with particularity in the claims that follow. A better understanding of the features and advantages of the present disclosure will be obtained by reference to the following detailed description that sets forth illustrative embodiments, in which the principles of the disclosure are utilized, and the accompanying drawings.
FIG. 1 is a diagrammatic cross section of a patient heart illustrating an example implantation of a modular biostimulator in a target anatomy, in accordance with an embodiment.
FIG. 2 is a perspective view of a modular biostimulator, in accordance with an embodiment.
FIG. 3 is an exploded view of a modular biostimulator, in accordance with an embodiment.
FIG. 4 is a flowchart of a method of implanting a modular biostimulator for pacing.
FIGS. 5-7 are perspective views of operations in implanting a modular biostimulator, in accordance with an embodiment.
FIG. 8 is an exploded view of a modular biostimulator, in accordance with an embodiment.
FIGS. 9-11 are perspective views of operations in implanting a modular biostimulator, in accordance with an embodiment.
FIG. 12 is an exploded view of a modular biostimulator, in accordance with an embodiment.
FIGS. 13-14 are perspective views of operations in implanting a modular biostimulator, in accordance with an embodiment.
FIG. 15 is a perspective view of a header module, in accordance with an embodiment.

### DETAILED DESCRIPTION

Embodiments describe a biostimulator and a biostimulator system for pacing, e.g., septal pacing. The biostimulator may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known devices and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along an axis of a biostimulator. Similarly, "proximal" may indicate a second direction opposite to the first direction. Such terms are provided to establish relative frames of reference, however, and are not intended to limit the use or orientation of a biostimulator to a specific configuration described in the various embodiments below.

In an aspect, a biostimulator includes a modular design. For example, the biostimulator can include a first module, e.g., a header module, having an electrode and a fixation element to engage a target tissue, and a second module, e.g., a housing module, having pacing circuitry used to deliver pacing signals to the electrode. The header module and the housing module are separable and connectable and, thus, the header module can be delivered to the target tissue apart from the housing module. When the header module is placed in the target tissue, the housing module can be delivered and connected to the header module. The engaged modules form the biostimulator, which can perform pacing of the target tissue. Accordingly, the modular biostimulator allows the header module to be more easily maneuvered into place and the housing module to be delivered to the anchored header module with less need to manipulate the housing module. A likelihood of interfering with adjacent structures of the heart by the housing module can therefore be reduced.

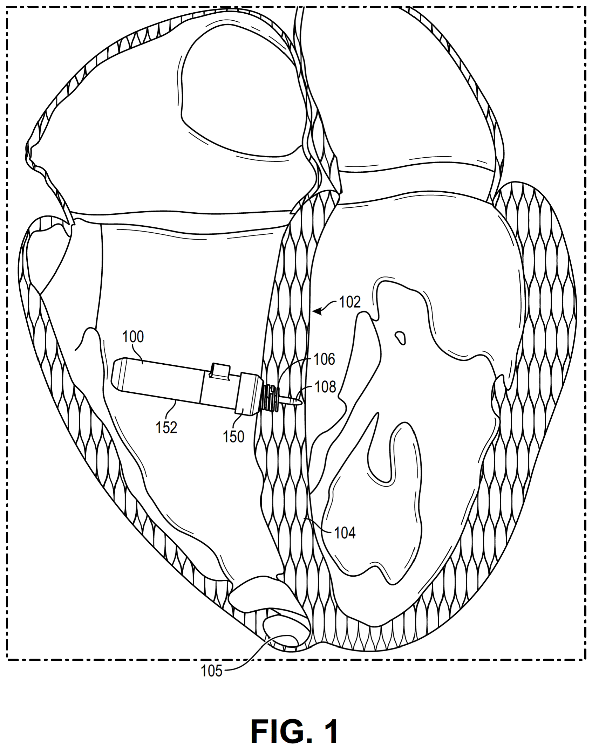

Referring to FIG. 1, a diagrammatic cross section of a patient heart illustrating an example implantation of a modular biostimulator in a target anatomy is shown in accordance with an embodiment. The diagram shows a biostimulator 100 attached to a patient heart 102. A leadless biostimulator system, e.g., a cardiac pacing system, can include the biostimulator 100. The biostimulator 100 can be implanted in the patient heart 102, and can be leadless (and thus, may be a leadless cardiac pacemaker). The biostimulator 100 can be placed in a cardiac chamber, such as a right atrium and/or right ventricle of the heart 102, or attached to an inside or outside of the cardiac chamber.

The biostimulator 100 can be modular, as described below. For example, the biostimulator 100 can include a header module 150 and a housing module 152. The header module 150 and/or housing module 152 can be attached to one or more of an interventricular septal wall 104 or a ventricular apex 105 of the heart 102. More particularly, the modules of the biostimulator 100 can be delivered to a ventricular chamber independently and then connected, electrically or mechanically, within the ventricular chamber. The header module 150 can be delivered to an interventricular septum, and one or more elements, such as a fixation element 106 and/or a pacing electrode 108, can pierce the interventricular septal wall 104 of the septum to engage and anchor the header module 150 to the tissue. The housing module 152 can be delivered into the ventricular chamber, and can be electrically and/or mechanically connected to the header module 150. Accordingly, the header module 150 can be maneuvered into place and affixed to the interventricular septal wall, and the housing module 152 can subsequently be attached to the header module 150 to anchor the housing module 152 to the interventricular septum.

When the biostimulator 100 is delivered to and screwed into the septum of the heart 102, the pacing electrode 108 may be positioned for deep septal pacing at a target anatomy, such as a bundle branch in the septum. For example, an active electrode of the pacing electrode 108 can be positioned at a left bundle branch in the septum. The biostimulator 100 may deliver pacing impulses through the pacing electrode 108 to the target anatomy. Accordingly, the pacing electrode 108 can be located to effectively probe and pace the target anatomy.

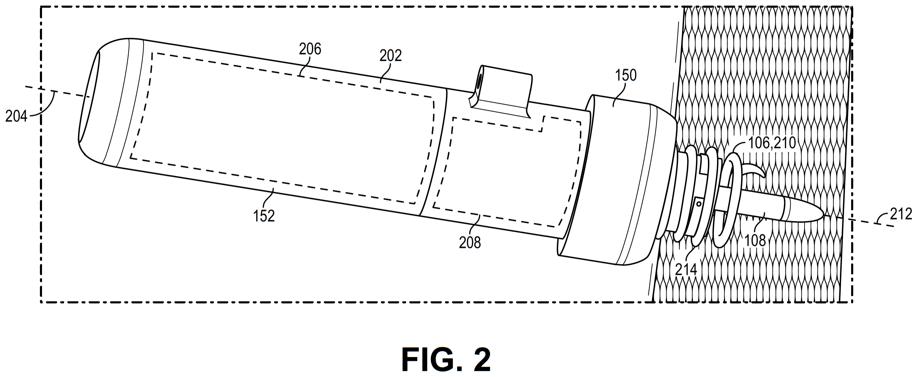

Referring to FIG. 2, a perspective view of a modular biostimulator is shown in accordance with an embodiment. The biostimulator 100 can be a leadless cardiac pacemaker that can perform cardiac pacing and that has many of the advantages of conventional cardiac pacemakers while extending performance, functionality, and operating characteristics. In a particular embodiment, the biostimulator 100 can use two or more electrodes located on or within a housing 202 of the biostimulator 100 for pacing the cardiac chamber upon receiving a triggering signal from at least one other device within the body. The two or more electrodes of the biostimulator 100 can include the pacing electrode 108 that acts as an active electrode and/or a portion of the housing 202 that acts as an active electrode. The electrodes can deliver pacing pulses to target anatomies, such as bundle branches within the septum of the heart 102, to perform pacing, e.g., deep septal pacing, and optionally, in some embodiments can sense electrical activity from the muscle. The electrodes may also communicate bidirectionally with at least one other device within or outside the body.

The housing 202 has a longitudinal axis, e.g., a housing axis 204. The housing axis 204 can be centrally located. For example, the housing 202 can have a cylindrical outer surface that is coaxially located with the housing axis. As described below, several biostimulator components can be located along the housing axis 204, and one or more components may be radially offset from the housing axis.

The housing 202 can contain a power source 206 (shown by hidden lines) to provide power for pacing, sensing, and communication, which may include, for example, bidirectional communication. The power source 206 may, for example, include a battery, an energy harvesting device, etc. The housing 110 can optionally contain an electronics compartment 208 (shown by hidden lines) to hold circuitry adapted for different functionality. For example, the electronics compartment 208 can contain pacing circuitry for sensing cardiac activity from the electrodes or for receiving information from at least one other device via the electrodes. The pacing circuitry may be electrically connected to the power source to deliver energy from the power source to a target anatomy. For example, the pacing circuitry may be used for generating pacing pulses for delivery to tissue via the pacing electrode 108.

The pacing circuitry can be electrically connected to the pacing electrode 108. The electronics compartment 208 may contain circuits for transmitting information to at least one other device via the electrodes and in some embodiments can optionally contain circuits for monitoring device health. The circuitry of the biostimulator 100 can control these operations in a predetermined manner. In some implementations of a cardiac pacing system, cardiac pacing is provided without a pulse generator located in the pectoral region or abdomen, without an electrode-lead separate from the pulse generator, without a communication coil or antenna, and without an additional requirement of battery power for transmitted communication.

In an embodiment, the fixation element 106 can fix the biostimulator 100 to an intracardial implant site, e.g., at the septal wall. More particularly, the biostimulator 100 may include one or more engaging mechanisms, such as the fixation element 106. The fixation element 106 can be an active fixation mechanism or a passive fixation mechanism. For example, the fixation element 106 can include a fixation helix, e.g., a helical anchor 210, or fixation tines. The fixation helix can extending helically about a fixation axis 212. More particularly, the helical anchor 210 can include a screw or helical member that screws into the target tissue. Passive fixation mechanisms can include prongs, for example, to engage affix to heart structures without penetrating tissue. The fixation element 106 can be connected to the housing 202, e.g., via a fixation mount 214 of the header module 150. For example, as described below, the header module 150 can be mechanically attached to a distal portion of the housing. Accordingly, when the fixation element 106 is implanted into a septal wall, the header module 150 can hold the housing 202 to anchor the housing module 152 against the septal wall.

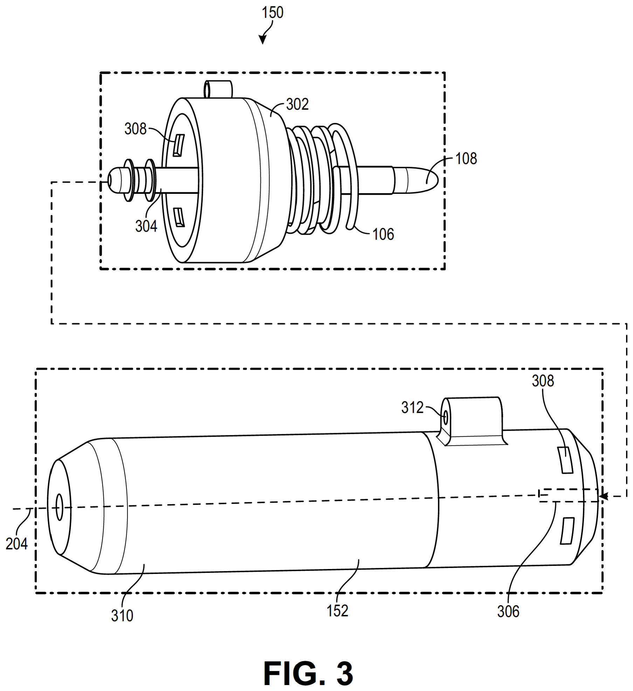

Referring to FIG. 3, an exploded view of a modular biostimulator 100 is shown in accordance with an embodiment. The modular biostimulator 100 includes the header module 150 that can be disconnected and separated from the housing module 152. The header module 150 can include features to attach to a target tissue. For example, as described above, the fixation element 106 can be mounted on a header body 302. The fixation element 106 can be secured to the header body 302 such that movement of the header body 302 transmits movement to the fixation element 106. Rotation of the header body 302 can transmit rotation to the fixation element 106 to cause the element to screw into the target tissue, for example.

The header module 150 can include features to deliver electrical impulses to the target tissue. In an embodiment, the header module 150 can include an electrical pin 304 electrically connected to the pacing electrode 108. More particularly, the electrical pin 304 can extend proximally from the header body 302 and the pacing electrode 108 can extend distally from the header body 302. The electrical pin 304 may be interconnected with the pacing electrode 108 through an electrical feedthrough located within the header body 302. Accordingly, electrical pulses delivered to the electrical pin 304 can transmit distally through the electrical feedthrough to the pacing electrode 108.

In an embodiment, the housing module 152 includes an electrical socket 306 to receive the electrical pin 304. The electrical socket 306 can have conductive surfaces sized and located to engage corresponding conductive surfaces of the electrical pin 304 when the pin is inserted into the socket. For example, the socket and the pin can engage each other through an electrical connection having one or more contacts and seals, such as an IS-1 electrical connector design. Accordingly, the electrical socket 306 can electrically connect to the electrical pin 304. The electrical socket 306 may be electrically connected to the pacing circuitry, e.g., to communicate electrical pulses to the pacing circuitry. For example, the pacing circuitry can generate pacing pulses that are communicated to the electrical socket 306. Accordingly, the electrical socket 306 can electrically connect the electrical pin 304 to the pacing circuitry such that the pacing pulses are transmitted distally through the pin and the pacing electrode 108 into the target tissue.

The modular biostimulator 100 can include features to mechanically connect the modules to each other. For example, an attachment mechanism 308 may be incorporated in one or more of the header module 150 or the housing module 152 to secure the modules. The attachment mechanism 308 can include complementary parts that engage to connect the modules and disengage to disconnect the modules. For example, one of the modules, e.g., the header module 150, can include one or more tabs. The tabs may, for example, extend from an interior surface of the header body 302 into a space within which the housing module 152 inserts when the modules are engaged. The other module, e.g., the housing module 152, can include one or more slots that complement the tab(s). For example, the slots may be recesses formed in a surface that apposes the interior surface of the header body 302 when the modules are engaged. The tab(s) can insert into the recess(es) when the modules are joined, forming a snap fit between the parts.

The tab and recess interconnection is provided by way of example and it will be appreciated that other modes of connecting the modules may be used. For example, the header body 302 can form a friction or press fit with an exterior surface of a housing body 310. Alternatively, detents can engage recesses in opposing surfaces to form a snap fit. The illustrated mode is therefore provided by way of example and not limitation. In any case, the attachment mechanism 308 can mechanically connect the header module 150 to the housing module 152 when the electrical pin 304 is inserted into the electrical socket 306 and the modules are pressed together.

The modules of the biostimulator 100 can be tracked toward the target anatomy. In an embodiment, the housing module 152 includes a guide lumen 312. The guide lumen 312 can be a channel to receive a tracking rail, such as a guidewire-like component of a transport system, as described below. The channel may be formed in a guide body, e.g., a collar surrounding the guide lumen 312. The guide lumen 312 can be sized to receive a guidewire that is placed within the target anatomy. Accordingly, the housing module 152 can be advanced along the guidewire by pushing the housing body 310 forward, as described below.

In an embodiment, the guide lumen 312 is radially offset from an axis of the housing module 152. For example, the housing axis 204 may be aligned with the electrical socket 306 that receives the electrical pin 304. Housing axis 204 can be central to the housing body 310. The guidewire that is used to track the housing module 152 through the anatomy may be radially offset from the electrical pin 304. Accordingly, the guide lumen 312, which receives the guidewire, may be similarly offset from the housing axis 204. The guide lumen 312 may be defined by a guide body extending radially outward from the housing body 310. The guide body can include a cylindrical collar having the guide lumen 312.

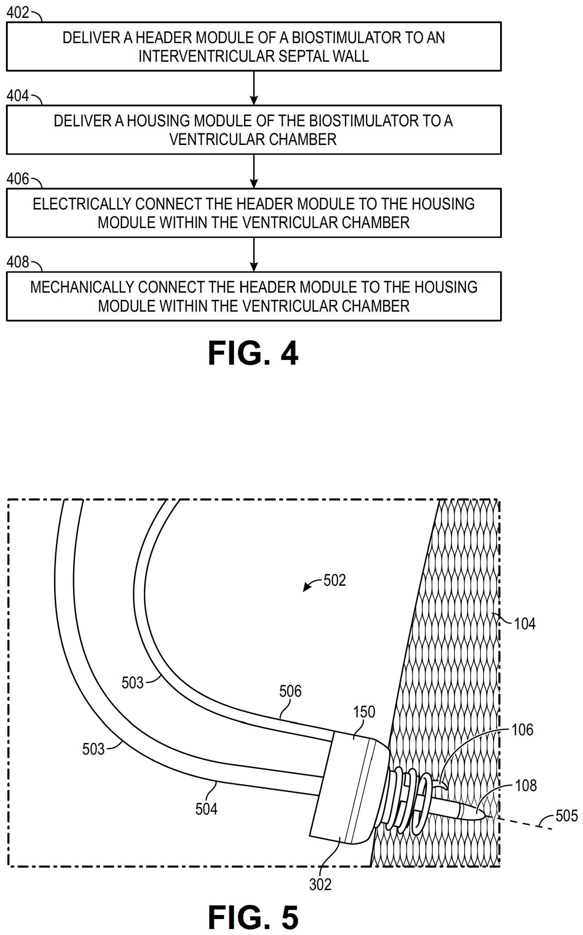

Referring to FIG. 4, a flowchart of a method of implanting a modular biostimulator for pacing is shown. Having introduced components of the modular biostimulator 100, an interaction and function of the components may be further understood with respect to the method of implanting the modular biostimulator 100. Operations of the method are shown in FIGS. 5-7. Accordingly, FIGS. 4-7 are described in combination below.

Referring to FIG. 5, a perspective view of an operation in a method of implanting a modular biostimulator is shown. Referring also to FIG. 4, in operation 402, the header module 150 of the biostimulator 100 is delivered to the interventricular septal wall 104. The modular biostimulator 100 can be part of a biostimulator system 502. More particularly, the biostimulator system 502 can include the modules of the biostimulator 100, e.g., the header module 150 and the housing module 152, and a biostimulator transport system 503. The biostimulator transport system 503 can be used to transport, e.g., deliver or retrieve, one or more of the modules to or from the septal wall 104.

The transport system can include guides, e.g., rails, shafts, catheters, etc., to track the modules through the target anatomy. In an embodiment, the biostimulator transport system 503 includes a guide shaft 504 to advance the header module 150 through the target anatomy. The guide shaft 504 can be connected to the header module 150. For example, the guide shaft 504 may include a catheter having a central lumen to receive the electrical pin 304. The electrical pin 304 can engage the catheter in a friction fit. Alternatively, the header module 150 may include a threaded portion, e.g., on an outer surface of the pin near the header body 302, to receive and engage a corresponding threaded portion of the guide shaft 504. The guide shaft 504 may therefore be mechanically connected to the header module 150.

The electrical pin 304 can be inserted into the lumen, and the catheter shaft may be manipulated at a proximal end to steer the header module 150 toward the septal wall 104. More particularly, the guide shaft 504 may be rotated, pushed, pulled, etc. to advance the pacing electrode 108 into the ventricular chamber. The header module 150 is compact, and thus, may be more easily steered into place within the limited available space of the ventricle. Accordingly, the pacing electrode 108 can be engaged to the septal wall 104 at an appropriate angle to reach the target anatomy for effective pacing.

When the pacing electrode 108 is placed at the septal wall 104, the guide shaft 504 can be pushed forward to plunge the pacing electrode 108 into the target tissue along the pacing axis 505. The guide shaft 504 may then be rotated to screw the fixation element 106 into the target tissue. The header module 150 may then be anchored in the target tissue, with the pacing electrode 108 located for tissue pacing.

Accurate placement of the pacing electrode 108 for tissue pacing may be monitored via the guide shaft 504. In an embodiment, the guide shaft 504 is electrically connected to the pacing electrode 108. For example, the guide shaft 504 may have electrical contacts that connect to the electrical pin 304 when the pin is located in the shaft lumen. Electronics located external to the ventricle, e.g., in an operating room, may be placed in communication with the electrical contacts through electrical connectors at a proximal end of the guide shaft 504. Electrical signals may therefore be transmitted to or from the pacing electrode 108 through the electrical pin 304 and the guide shaft 504. Afferent signals may be used to pace the target tissue, e.g., through test paces. Efferent signals may be received by the external electronics for analysis. Placement of the housing module 152 may continue until an acceptable pacing location is achieved. When the pacing electrode 108 is located for effective pacing, the housing module 152 may be advanced into place to connect to the header module 150, as described below.

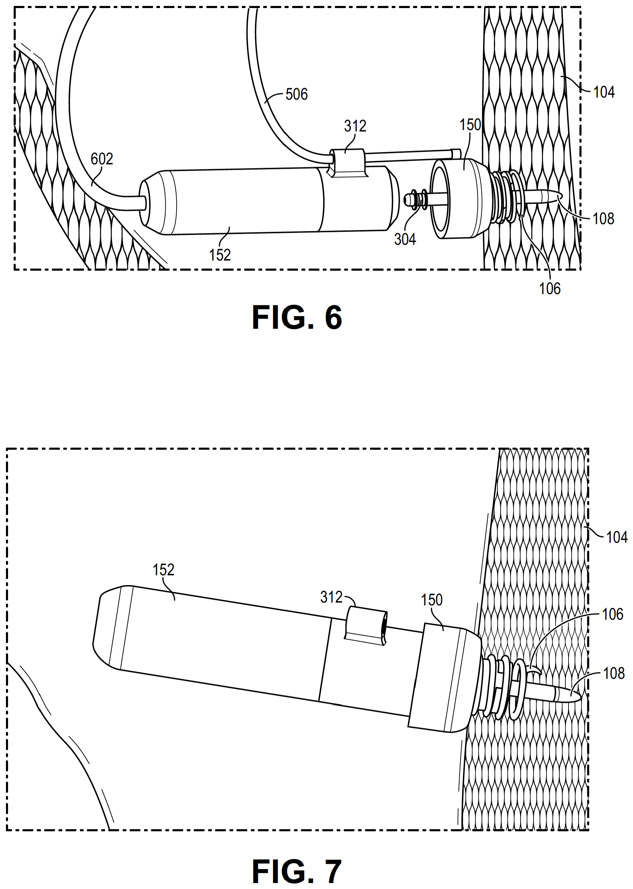

Referring to FIG. 6, a perspective view of an operation in a method of implanting a modular biostimulator is shown. Also referring to FIG. 4, at operation 404, the housing module 152 is delivered to the ventricular chamber. In an embodiment, biostimulator transport system 503 includes a guide rail 506 over which the housing module 152 may be tracked. The guide rail 506 may include a flexible wire. The wire may be elongated and have a proximal end and a distal end. The distal end can connect to the header module 150. For example, the header module 150 can have a channel or a groove within which the distal end of the guide rail 506 may be lodged. Guide rail 506 can form a friction fit within the channel of the header module 150. Accordingly, the guide rail 506 may resist removal from the header module 150, however, the guide rail 506 may be dislodged from the header module 150 when sufficient pulling force is applied. Alternatively, the mechanical connection between the guide rail 506 and the header module 150 may include a threaded connection. For example, the channel in the header module 150 may be threaded, and can engage a corresponding thread on the distal end of the guide rail 506. The guide rail 506 can accordingly be removed by rotating the guide rail 506 to unscrew the threads.

The guide rail 506 can guide the housing module 152 toward the header module 150. As described above, the guide rail 506 may be connected to the header module 150. The guide rail 506 can extend through the guide lumen 312 of the housing module 152. The housing module 152 can therefore be tracked over the guide rail 506. The guide rail 506 can act as a guidewire to lead the housing module 152 toward the header module 150.

Advancement of the housing module 152 over the guide rail 506 may be driven by a housing guide shaft 602. The housing guide shaft 602, like the guide shaft 504, may include a catheter, tubular structure, or wire having an elongated body. The elongated body can engage a proximal end of the housing module 152. For example, a distal end of the housing guide shaft 602 can insert into a recess formed in a proximal face of the housing module 152. The housing guide shaft 602 can engage the recess in a friction fit, or a threaded connection. Accordingly, the housing guide shaft 602 can be pushed, pulled, or torqued to steer the housing module 152 along the guide rail 506 toward the header module 150. The housing guide shaft 602 can steer the housing module 152 along the guide rail 506 in an over-the-wire manner.

Delivering the housing module 152 over the guide rail 506 can steer the housing module 152 into place without requiring substantial steering of the housing module 152. For example, the housing module 152 may be rotated or spun about the guide rail 506. Tilting of the housing module 152 may be unnecessary, however, because the guide rail 506 can bring the housing module 152 to the implantation site at the correct angle determined by the header module 150. Accordingly, the housing module 152 can be properly placed despite the limited available space and range of motion available within the upper region of the ventricle.

At operation 406, the header module 150 is electrically connected to the housing module 152. the electrical connection can be a wet connection, e.g., made in vivo within the blood in the ventricular chamber. The housing guide shaft 602 can have sufficient stiffness to push the housing module 152 forward over the guide rail 506. When a distal end of the housing body 310 approaches the electrical pin 304, the housing guide shaft 602 can be manipulated to align the electrical socket 306 within the housing module 152 to the electrical pin 304. The housing module 152 can be pushed to advance the electrical socket 306 over the electrical pin 304 in order to make an electrical connection between the pacing circuitry within the housing module 152 and the pacing electrode 108 that is engaged with the target tissue. The electrical connection may be protected by seals on the pin or within the socket. The seals can displace blood or other body fluids when the connection is made to reduce a likelihood of incursion of fluid into the electrical connection.

Referring to FIG. 7, a perspective view of an operation in a method of implanting a modular biostimulator is shown. Also referring to FIG. 4, at operation 408, the header module 150 is mechanically connected to the housing module 152 within the ventricular chamber. The mechanical connection can be provided by the attachment mechanism 308 described above. For example, tabs of the header module 150 may engage corresponding recesses in the housing module 152. The tabs can snap into place to connect the modules together and resist their separation. Alternative forms of interconnection, such as friction fits, threaded connections, etc. may also be used to mechanically connect housing module 152 to the header module 150.

The guide components can be removed from the modular biostimulator 100 to release the anchored components and complete implantation. For example, the guide rail 506 may be pulled, rotated, or otherwise manipulated to mechanically disconnect the guide shaft 504 from the header module 150. Similarly, the housing guide shaft 602 may be manipulated to mechanically disconnect the housing guide shaft 602 from the housing module 152. The guide rail 506 can be retracted through the guide lumen 312 of the housing module 152. When the distal ends of the guide components are detached from the modular biostimulator 100, the guide components can be pulled and removed from the target anatomy. By contrast, the modular biostimulator 100 may remain intact and in place at the target tissue. The pacing electrode 108 can be located at the target site, and pacing pulses may be delivered from the pacing circuitry of the housing module 152 through the header module 150 to the pacing site for ongoing electrical stimulation.

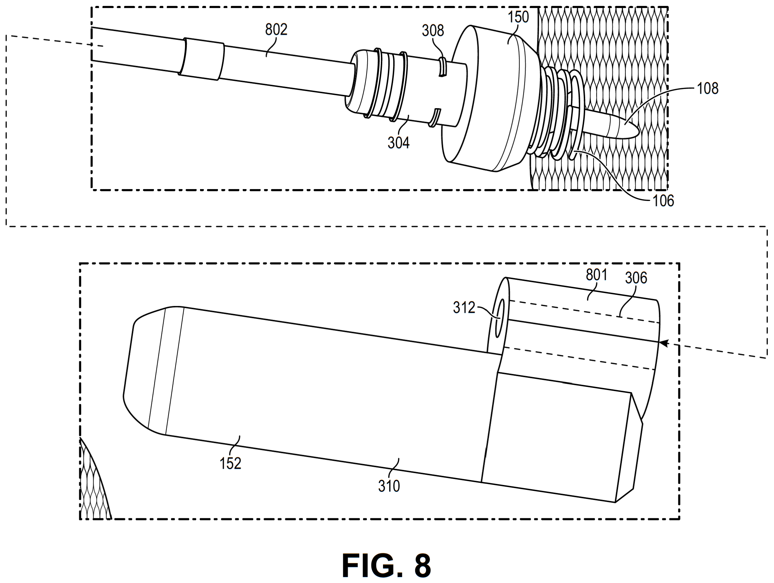

Referring to FIG. 8, an exploded view of a modular biostimulator is shown in accordance with an embodiment. Components of the modular biostimulator 100 shown in FIG. 8 can be similar to the components of the modular biostimulator 100 shown in FIG. 3. For the sake of brevity, such components are not described again here, however, it will be appreciated that the description above may apply to the corresponding components of both figures.

The modular biostimulator 100 may, as described above, include the electrical socket 306 centrally located, e.g., along the housing axis 204. The electrical socket 306 may, however, be located differently. The housing module 152 may include a shoulder 801 extending laterally outward from the housing body 310. The shoulder 801 can be radially offset from the housing body 310. Furthermore, the guide lumen 312 may extend through the shoulder 801, e.g., laterally offset from the housing body 310 having the pacing circuitry and the power source 206. In an embodiment, the electrical socket 306 may be located within the guide lumen 312. For example, the guide lumen 312 of the housing module 152, which can receive the guide rail 506 of the biostimulator transport system 503, may have electrical connections internally to engage the electrical pin 304 of the header module 150. Furthermore, the electrical pin 304 and/or the electrical socket 306 can include seals to displace blood, as described above. Accordingly, the electrical pin 304 of the header module 150 can be inserted into the electrical socket 306 of the housing module 152 to form a wet connection within the ventricle.

The header module 150 may include a delivery prong 802 extending proximally from the electrical pin 304. The delivery prong 802 provides an extension to which a guide component can attach. For example, the guide shaft 504 may mount over the delivery prong 802 to manipulate the header module 150 during delivery. The delivery prong 802 can include an electrical contact to engage a corresponding electrical contact of the guide shaft 504. Accordingly, pacing and monitoring of the target tissue may be performed through the delivery prong 802 during delivery.

Pacing circuitry within the housing module 152 may electrically communicate with electrical contacts in the guide lumen 312. For example, wires, vias, or other electrical connectors can extend laterally from the pacing circuitry within the housing body 310 to the electrical contacts within the guide lumen 312. Accordingly, when the electrical pin 304 is located within the guide lumen 312 and electrical connection is made between the header module 150 and the housing module 152, the pacing circuitry can deliver pacing pulses through the header module 150 to pacing electrode 108.

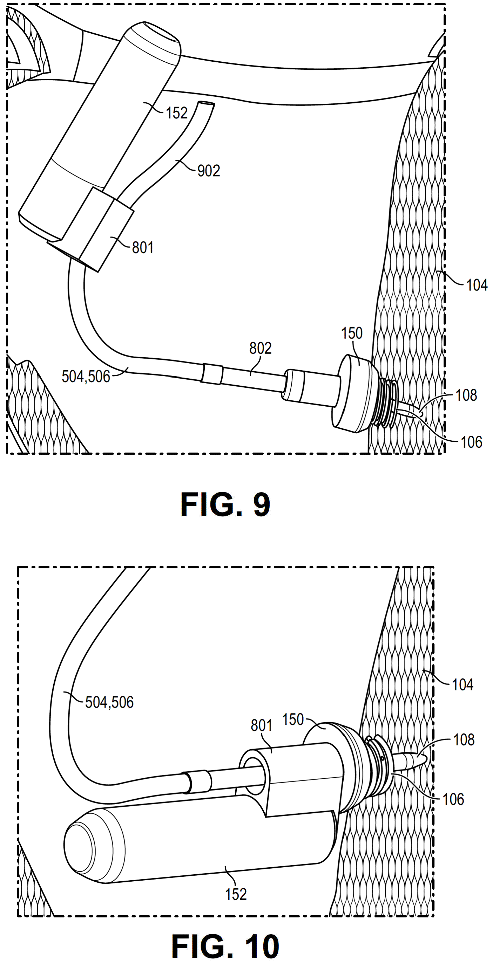

Referring to FIG. 9, a perspective view of an operation in a method of implanting a modular biostimulator is shown. The implantation method described above with respect to FIG. 4 can be applied to any of the modular biostimulator 100 embodiments described herein. At operation 402, the header module 150 can be advanced to the interventricular septal wall 104. Advancement of the header module 150 can be urged by a guide shaft 504 connected to the delivery prong 802. More particularly, the guide shaft 504 may receive the delivery prong 802, and a user may push forward on the guide shaft 504 to advance the header module 150. The header module 150 can be steered to the septal wall 104. When located at the septal wall 104, the header module 150 can be pushed and/or rotated to plunge the pacing electrode 108 into the target tissue and to anchor the header module 150 within the septal wall 104. As described above, the guide shaft 504 can be used to sense and/or pace through the header module 150 to confirm appropriate placement of the pacing electrode 108. When the pacing electrode 108 is appropriately placed, the housing module 152 may be delivered toward the header module 150.

In an embodiment, tracking the housing module 152 to the ventricular chamber at operation 404 includes advancing a guide catheter 902 over the guide rail 506. In the embodiment shown in FIG. 9, the guide rail 506 may be the guide shaft 504. More particularly, the guide component used to steer the header module 150 may also be the guide component used to track the housing module 152. The guide rail 506 may extend from the delivery prong 802 through the guide lumen 312 of the housing module 152. Accordingly, when the guide rail 506 is loaded through the guide lumen 312, the guide catheter 902 may be tracked over the guide rail 506 to push the housing module 152 toward the header module 150.

The guide catheter 902 can have a central lumen sized to receive the guide rail 506. An outer diameter of the guide catheter 902, however, may be larger than an inner dimension of the guide lumen 312. Accordingly, a distal tip of the guide catheter 902 can interfere with and engage a proximal face of the portion of the housing module 152 having the guide lumen 312. More particularly, the distal tip can be pressed against the shoulder 801. The housing module 152 may thereby be advanced to the header module 150 over the guide rail 506.

Referring to FIG. 10, a perspective view of an operation in a method of implanting a modular biostimulator is shown. Also referring to FIGS. 3, 4, and 9, the guide catheter 902 can be pushed forward until the housing module 152 is advanced fully over the guide rail 506. The housing module 152 can advance over the delivery prong 802 onto the electrical pin 304. More particularly, the electrical pin 304 can make electrical contact with the electrical socket 306. Accordingly, the header module 150 can be electrically connected to the housing module 152 within the ventricular chamber at operation 406.

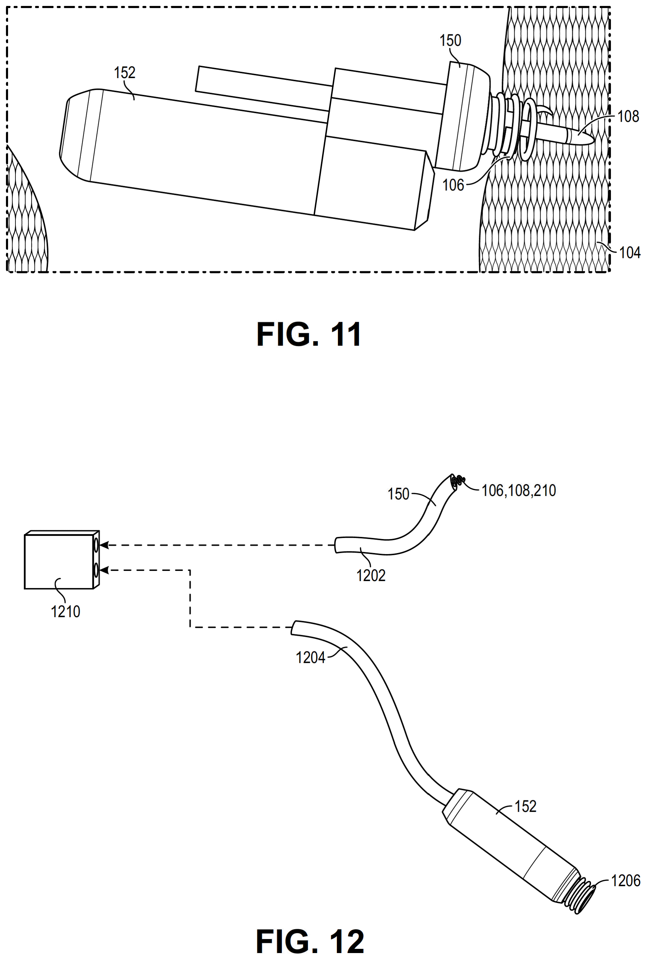

Referring to FIG. 11, a perspective view of an operation in a method of implanting a modular biostimulator is shown. Also referring to FIGS. 4 and 8, in addition to being electrically connected, the header module 150 and the housing module 152 can be mechanically connected, at operation 408, at the implant site. For example, as described above, the header module 150 can snap, slip, or thread into place within the housing module 152 to form the modular biostimulator 100. When mechanically connected, the seals within the shoulder 801 can protect the electrical connection between the modules. Guide components, such as the guide rail 506 and the guide catheter 902, can be removed from the corresponding implant components and from the target anatomy. Accordingly, modular biostimulator 100 may be left in place to allow electrical pacing to be delivered through the pacing electrode 108 to the septal wall 104.

Referring to FIG. 12, an exploded view of a modular biostimulator is shown in accordance with an embodiment. Components of the modular biostimulator 100 shown in FIG. 12 can be similar to the components of the modular biostimulator 100 shown in FIG. 3. For example, the housing module 152 can include pacing circuitry, and the header module 150 can include the fixation element 106, as described above. For the sake of brevity, such components are not described again here, however, it will be appreciated that the description above may apply to the corresponding components of both figures.

Despite having similarities with the embodiments described above, the modular biostimulator 100 shown in FIG. 12 can also have differences. The header module 150 can include a header leadlet 1202, or short conductive cable. Here, the term short can mean having a length less than a distance from a tricuspid valve to a ventricular apex 105 of a ventricle. The header leadlet 1202 can be electrically connected to the pacing electrode 108. In an embodiment, the pacing electrode 108 can be part of the same structure that forms the fixation element 106. For example, the fixation element 106 can be a helical anchor 210 formed from conductive material. The helical anchor 210 can electrically connect to a wire extending longitudinally through the header leadlet 1202. The wire can be surrounded by an insulative sheath. Accordingly, electrical pulses delivered to the wire can transmit distally through the fixation element 106, which includes the pacing electrode 108, into target tissue.

The housing module 152, like the header module 150, can include a housing leadlet 1204, or short conductive cable. The housing leadlet 1204 may be electrically connected to the pacing circuitry within the housing body 310. For example, the housing leadlet 1204 may include a wire extending longitudinally through the leadlet from a proximal end into the housing body 310. The wire can connect to the pacing circuitry to receive pacing pulses generated by the circuitry. The wire can be surrounded by an insulative sheath.

The housing module 152 may include an anchor element 1206. The anchor element 1206 may include a helical anchor 210, tines, or other fixation features. The anchor element 1206 may be used to anchor the housing module 152 to a non-pacing site within the ventricle. For example, the anchor element 1206 may be anchored at a ventricular apex 105. Anchoring the housing module 152 at a non-pacing site can separate the housing module 152 from sensitive structures near the pacing site, such as valve leaflets or an opposite heart 102 wall.

The biostimulator modules may be interconnected in vivo. In an embodiment, the biostimulator 100 includes a cable connector 1210. The cable connector 1210 can include one or more channels to receive the module leadlets. For example, a proximal end of the header leadlet 1202 may be received in a first channel of the cable connector 1210, and a proximal end of the housing leadlet 1204 may be received in a second channel of the cable connector 1210. When received within the cable connector 1210, an electrical connection may be made between the leadlets. For example, the cable connector 1210 can include a blade or another conductive interconnect that can plunge into, squeeze, or otherwise make contact with the wires inside of the leadlets. The conductive interconnect may therefore electrically connect the internal wires of the leadlets and, thus, can place the pacing circuitry in electrical connection with the pacing electrode 108.

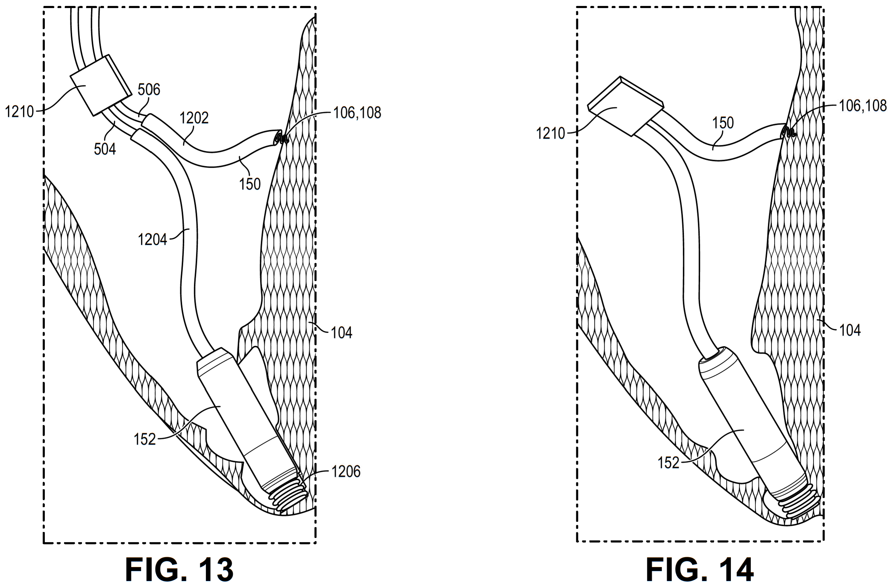

Referring to FIG. 13, a perspective view of an operation in a method of implanting a modular biostimulator is shown. The implantation method described above with respect to FIG. 4 can be applied to the modular biostimulator 100 embodiment of FIGS. 12 and 13. At operation 402, the header module 150 can be advanced to the interventricular septal wall 104. The header module 150 may be steered into place by the guide rail 506. For example, the guide rail 506 can connect to the proximal end of the header leadlet 1202, and may be pushed, pulled, or twisted to steer the header module 150 toward the septal wall 104. When located at the septal wall 104, the header module 150 may be pushed and/or rotated to anchor the fixation element 106 and the pacing electrode 108 within the septal wall 104.

At operation 404, the housing module 152 can be delivered to the ventricular chamber. The guide sheath can be attached to the proximal end of the housing leadlet 1204. The guide sheath can be pushed, pulled, or rotated to steer the housing module 152 to the non-pacing site. For example, the housing module 152 can be steered downward into the ventricular apex 105. The anchor element 1206 may therefore, optionally, be connected, actively or passively, to the apex or the heart structures in a vicinity of the apex. Active fixation can refer to puncture of tissue by an active fixation element 106, such as a helical anchor or tines. Passive fixation can refer to interference with tissue by a passive fixation element, such as prongs. The housing module 152 may therefore be retained within the ventricle out of the way of sensitive heart structures.

At operation 406, the biostimulator modules can be electrically connected to each other. In an embodiment, electrically coupling the header module 150 to the housing module 152 includes interconnecting the header leadlet 1202 and the housing leadlet 1204 by the cable connector 1210. The cable connector 1210 can be advanced over the guidewire rail and the guide shaft 504. More particularly, the guide components can extend through the channels in the cable connector 1210. The guide components may therefore guide the cable connector 1210 toward the leadlets. One or more guide or backing catheters (not shown) can be tracked over the guide components to push the cable connector 1210 forward. For example, the guide catheter(s) can have respective internal lumen(s) to receive the guide components and the distal tip of the catheter(s) can be pressed against the cable connector 1210 to urge it forward into the ventricle and over the leadlets 1202, 1204.

When the leadlets 1202, 1204 are received within the channels of the cable connector 1210, the conductive interconnect can be actuated to engage the wires of the leadlets and place the leadlets 1202, 1204 in electrical communication with each other. The cable connector 1210 may include internal seals to protect the electrical connection between the leadlets 1202, 1204 and provide a wet connection, as described above. When the leadlets 1202, 1204 are electrically connected, pacing impulses can be delivered from the housing module 152 to the header module 150.

Referring to FIG. 14, a perspective view of an operation in a method of implanting a modular biostimulator 100 is shown. Also referring to FIG. 4, delivery of the cable connector 1210 onto the header leadlet 1202 and the housing leadlet 1204 within the ventricular chamber can provide mechanical and connection between the modules at operation 408. When the cable connector 1210 is delivered onto the leadlets 1202, 1204, the guide components can be removed. For example, the guide rail 506 and the guide shaft 504 can be disconnected from the leadlets 1202, 1204. Similarly, the guide or backing catheters can be retracted away from the cable connector 1210. The guide connectors can be fully removed from the anatomy, leaving the modular biostimulator 100 in place. The modular biostimulator 100 can be implanted within the ventricle. The fixation element 106 and pacing electrode 108 may be engaged with the septal wall 104. The housing module 152 located at the non-pacing site can deliver pacing impulses through the cable connector 1210 to the pacing electrode 108 to perform electrical stimulation of the target tissue by the modular biostimulator 100.

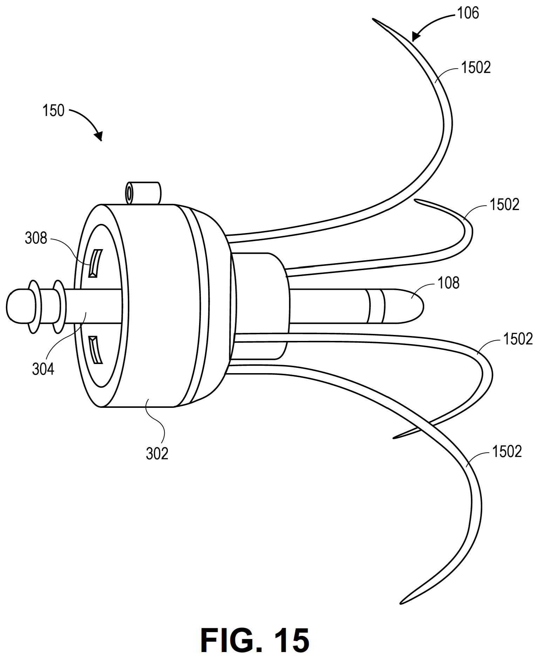

Referring to FIG. 15, a perspective view of a header module is shown in accordance with an embodiment. As described above, the modular biostimulator 100 includes the header module 150 that can be disconnected and separated from the housing module 152. The header module 150 can include features to attach to a target tissue. For example, the fixation element 106 can be mounted on and coupled to the header body 302 such that movement of the header body 302 transmits movement to the fixation element 106.

In an embodiment, the fixation element 106 includes one or more tines 1502. The tines 1502 may, for example, include elongated prongs that extend distally from the header body 302 to respective tine tips. The slender projections of the tines may curve between the header body 302 and the tine tips. For example, each tine, in a deployed state, may extend distally to an apex and curve from the apex to the tine tip at a location proximal to the apex. In an undeployed state, the tines may be straightened, e.g., within a delivery sheath, and the slender bodies of the tines can extend distally from the header body 302 to the tine tip at a location distal to the region of the tine that will become the apex in the deployed state. More particularly, the tines 1502 may be resiliently deformed into the straightened, undeployed state and when the tines are exposed from the protective sheath, the tines may recover to the curved, deployed state, as shown in FIG. 15. Accordingly, the tines may be advanced into the target tissue from the delivery sheath and then curve backward to capture the tissue and retain the pacing electrode against the target tissue. The tine structure shown in FIG. 15 may be incorporated into any of the fixation element embodiments described above.

## Claims

1. A biostimulator (100), comprising:
a housing module (152) including pacing circuitry electrically coupled to a power source (206), and a housing leadlet (1204) electrically coupled to the pacing circuitry;
a header module (150) including a pacing electrode (108), and a header leadlet (1202) electrically coupled to the pacing electrode (108); and
a cable connector (1210) interconnecting the housing leadlet (1204) and the header leadlet (1202), **characterized in that** the cable connector (1210) is configured to provide a wet connection between the housing module (152) and the header module (150).

2. The biostimulator of claim 1, wherein the wet connection is made in vivo within blood.

3. The biostimulator of claim 1 or 2, wherein the cable connector (1210) includes one or more channels configured to receive the housing leadlet (1204) electrically connected to the pacing circuitry and the header leadlet (1202) electrically connected to the pacing electrode (108).

4. The biostimulator of claim 3, wherein a proximal end of the housing leadlet (1204) is received in a first channel of the cable connector (1210), and wherein a proximal end of the header leadlet (1202) is received in a second channel of the cable connector (1210).

5. The biostimulator of any one of claims 1 to 4, wherein the cable connector (1210) includes a conductive interconnect configured to engage respective wires of the housing leadlet (1204) and the header leadlet (1210).

6. The biostimulator of claim 5, wherein the conductive interconnect is configured to plunge into or squeeze the respective wires.

7. The biostimulator of claim 5 or 6, wherein the conductive interconnect (1210) includes a blade.

8. The biostimulator of any one of claims 1 to 7, wherein the cable connector (1210) includes internal seals to provide a wet connection between the housing leadlet (1204) and the header leadlet (1202).

9. The biostimulator of claim 1, wherein the header module (150) includes a fixation element (106).

10. The biostimulator of claim 9, wherein the fixation element (106) includes a conductive helical anchor (210).

11. The biostimulator of any one of claims 1 to 10, wherein the housing module (152) includes an anchor element (1206).

12. The biostimulator of claim 11, wherein the anchor element (1206) includes a helical anchor (210).

13. A biostimulator system, comprising:
a biostimulator transport system (503); and
the biostimulator (100) of any one of claims 1 to 12 mounted on the biostimulator transport system (503).

14. The biostimulator system of claim 13, wherein the biostimulator transport system (503) includes one or more guide components comprising:
a guide rail (506) coupled to the header module (150); and/or
a guide sheath (504) coupled to the housing module (150).

15. The biostimulator system of claim 14, wherein the one or more guide components extend through one or more channels in the cable connector (1210).

## Patentansprüche

1. Biostimulator (100), umfassend:
ein Gehäusemodul (152), das eine Schrittmacherschaltung, die elektrisch an eine Leistungsquelle (206) gekoppelt ist, und einen Gehäuseleiterabgang (1204), der elektrisch an die Schrittmacherschaltung gekoppelt ist, beinhaltet;
ein Kopfteilmodul (150), das eine Schrittmacherelektrode (108) und einen Kopfteilleiterabgang (1202), der elektrisch mit der Schrittmacherelektrode (108) gekoppelt ist, beinhaltet; und
einen Kabelverbinder (1210), der den Gehäuseleiterabgang (1204) und den Kopfteilleiterabgang (1202) miteinander verbindet, **dadurch gekennzeichnet, dass** der Kabelverbinder (1210) dazu konfiguriert ist, eine Nassverbindung zwischen dem Gehäusemodul (152) und dem Kopfteilmodul (150) bereitzustellen.

2. Biostimulator nach Anspruch 1, wobei die Nassverbindung in vivo innerhalb von Blut hergestellt wird.

3. Biostimulator nach Anspruch 1 oder 2, wobei der Kabelverbinder (1210) einen oder mehrere Kanäle beinhaltet, die dazu konfiguriert sind, den Gehäuseleiterabgang (1204), der elektrisch mit der Schrittmacherschaltung verbunden ist, und den Kopfteilleiterabgang (1202), der elektrisch mit der Schrittmacherelektrode (108) verbunden ist, aufzunehmen.

4. Biostimulator nach Anspruch 3, wobei ein proximales Ende des Gehäuseleiterabgangs (1204) in einem ersten Kanal des Kabelverbinders (1210) aufgenommen wird und wobei ein proximales Ende des Kopfteilleiterabgangs (1202) in einem zweiten Kanal des Kabelverbinders (1210) aufgenommen wird.

5. Biostimulator nach einem der Ansprüche 1 bis 4, wobei der Kabelverbinder (1210) eine leitende Zwischenverbindung beinhaltet, die dazu konfiguriert ist, jeweilige Drähte des Gehäuseleiterabgangs (1204) und des Kopfteilleiterabgang (1210) in Eingriff zu nehmen.

6. Biostimulator nach Anspruch 5, wobei die leitende Zusammenverbindung dazu konfiguriert ist, in die jeweiligen Drähte einzusinken oder diese zusammenzudrücken.

7. Biostimulator nach Anspruch 5 oder 6, wobei die leitende Zwischenverbindung (1210) eine Klinge beinhaltet.

8. Biostimulator nach einem der Ansprüche 1 bis 7, wobei der Kabelverbinder (1210) interne Dichtungen beinhaltet, um eine Nassverbindung zwischen dem Gehäuseleiterabgang (1204) und dem Kopfteilleiterabgang (1202) bereitzustellen.

9. Biostimulator nach Anspruch 1, wobei das Kopfteilmodul (150) ein Befestigungselement (106) beinhaltet.

10. Biostimulator nach Anspruch 9, wobei das Befestigungselement (106) einen leitenden Schraubanker (210) beinhaltet.

11. Biostimulator nach einem der Ansprüche 1 bis 10, wobei das Gehäusemodul (152) ein Ankerelement (1206) beinhaltet.

12. Biostimulator nach Anspruch 11, wobei das Ankerelement (1206) einen Schraubanker (210) beinhaltet.

13. Biostimulatorsystem, umfassend:
ein Biostimulatortransportsystem (503); und
den Biostimulator (100) nach einem der Ansprüche 1 bis 12, der an dem Biostimulatortransportsystem (503) montiert ist.

14. Biostimulatorsystem nach Anspruch 13, wobei das Biostimulatortransportsystem (503) eine oder mehrere Führungskomponenten beinhaltet, die umfassen:
eine Führungsschiene (506), die mit dem Kopfteilmodul (150) gekoppelt ist; und/oder
eine Führungshülse (504), die mit dem Gehäusemodul (150) gekoppelt ist.

15. Biostimulatorsystem nach Anspruch 14, wobei sich die eine oder die mehreren Führungskomponenten durch einen oder mehrere Kanäle in dem Kabelverbinder (1210) erstrecken.

## Revendications

1. Biostimulateur (100), comprenant :
un module de boîtier (152) incluant une circuiterie de stimulation couplée électriquement à une source d'énergie (206), et un fil de boîtier (1204) couplé électriquement à la circuiterie de stimulation ;
un module d'embase (150) incluant une électrode de stimulation (108), et un fil d'embase (1202) couplé électriquement à l'électrode de stimulation (108) ; et
un connecteur de câble (1210) interconnectant le fil de boîtier (1204) et le fil d'embase (1202), **caractérisé en ce que** le connecteur de câble (1210) est configuré pour la fourniture d'une connexion humide entre le module de boîtier (152) et le module d'embase (150).

2. Biostimulateur selon la revendication 1, dans lequel la connexion humide est réalisée in vivo dans du sang.

3. Biostimulateur selon la revendication 1 ou 2, dans lequel le connecteur de câble (1210) inclut un ou plusieurs canaux configurés pour la réception du fil de boîtier (1204) connecté électriquement à la circuiterie de stimulation et du fil d'embase (1202) connecté électriquement à l'électrode de stimulation (108).

4. Biostimulateur selon la revendication 3, dans lequel une extrémité proximale du fil de boîtier (1204) est reçue dans un premier canal du connecteur de câble (1210), et dans lequel une extrémité proximale du fil d'embase (1202) est reçue dans un second canal du connecteur de câble (1210).

5. Biostimulateur selon l'une quelconque des revendications 1 à 4, dans lequel le connecteur de câble (1210) inclut une interconnexion conductrice configurée pour venir en prise avec des fils respectifs du fil de boîtier (1204) et du fil d'embase (1210).

6. Biostimulateur selon la revendication 5, dans lequel l'interconnexion conductrice est configurée pour plonger dans ou serrer les fils respectifs.

7. Biostimulateur selon la revendication 5 ou 6, dans lequel l'interconnexion conductrice (1210) inclut une lame.

8. Biostimulateur selon l'une quelconque des revendications 1 à 7, dans lequel le connecteur de câble (1210) inclut des joints d'étanchéité internes pour la fourniture d'une connexion humide entre le fil de boîtier (1204) et le fil d'embase (1202).

9. Biostimulateur selon la revendication 1, dans lequel le module d'embase (150) inclut un élément de fixation (106).

10. Biostimulateur selon la revendication 9, dans lequel l'élément de fixation (106) inclut un ancrage hélicoïdal conducteur (210).

11. Biostimulateur selon l'une quelconque des revendications 1 à 10, dans lequel le module de boîtier (152) inclut un élément d'ancrage (1206).

12. Biostimulateur selon la revendication 11, dans lequel l'élément d'ancrage (1206) inclut un ancrage hélicoïdal (210).

13. Système de biostimulateur, comprenant :
un système de transport de biostimulateur (503) ; et
le biostimulateur (100) selon l'une quelconque des revendications 1 à 12 monté sur le système de transport de biostimulateur (503).

14. Système de biostimulateur selon la revendication 13, dans lequel le système de transport de biostimulateur (503) inclut un ou plusieurs composants de guidage comprenant :
un rail de guidage (506) couplé au module d'embase (150) ; et/ou
une gaine de guidage (504) couplée au module de boîtier (150).

15. Système de biostimulateur selon la revendication 14, dans lequel l'un ou plusieurs composants de guidage s'étendent à travers un ou plusieurs canaux dans le connecteur de câble (1210).
